## Europäisches Patentamt
## European Patent Office
## Office européen des brevets

(19)

(11) Publication number : **0 486 277 B1**

(12)

# EUROPEAN PATENT SPECIFICATION

(45) Date of publication of patent specification :
10.11.93 Bulletin 93/45

(51) Int. Cl.⁵ : **B01J 31/10,** C08F 12/08,
C07C 37/20

(21) Application number : 91310471.7

(22) Date of filing : 13.11.91

(54) Process for catalyzing condensation reactions.

(30) Priority : 16.11.90 US 614346

(43) Date of publication of application :
20.05.92 Bulletin 92/21

(45) Publication of the grant of the patent :
10.11.93 Bulletin 93/45

(84) Designated Contracting States :
AT BE CH DE DK ES FR GB GR IT LI LU NL SE

(56) References cited :
EP-A- 0 268 866
GB-A- 1 023 426
US-A- 2 366 007
US-A- 3 965 039
US-A- 4 628 127

(73) Proprietor : ROHM AND HAAS COMPANY
Independence Mall West
Philadelphia Pennsylvania 19105 (US)

(72) Inventor : Lundquist, Eric Gustave
1915 Polo Run
Yardley, Pennsylvania 19067 (US)

(74) Representative : Tanner, James Percival et al
ROHM AND HAAS (UK) LTD. European
Operations Patent Department Lennig House
2 Mason's Avenue
Croydon CR9 3NB (GB)

## Description

The present invention is concerned with a process for catalyzing condensation reactions, and more particularly to a process for catalyzing condensation reactions using, as catalyst, strongly acidic, cation-exchange resin beads prepared from uniformly sized copolymer beads.

Strongly acidic ion-exchange resins may replace mineral acids such as sulfuric acid and hydrochloric acid in catalyzing reactions, for example condensation reactions. The use of the solid, acidic materials permits easier separation of the desired product from the catalyst in the reaction mixture, decreases equipment corrosion and complexity, and increases product purity. The use of the strongly acidic ion-exchange resins for catalyzing reactions is disclosed broadly in U.S.-A-3,037,052 to Bortnick, and the use of strongly acidic ion-exchange resins, prepared by sulfonating copolymers of styrene and polyethylenically unsaturated aromatic crosslinking monomers, as catalysts for the condensation of phenols with ketones or aldehydes to produce bisphenols, is disclosed in U.S.-A-3,153,001 to Apel *et al.,* U.S.-A-3,172,916 to Wagner, U.S.-A-3,634,341 to Gammill *et al.,* U.S.-A-4,590,303 and U.S.-A-4,391,997 to Mendiratta, U.S.-A-4,424,283 to Faler *et al.,* and others. Of particular interest is their use to catalyze the condensation of phenol with acetone to produce Bisphenol-A (para, para'-isopropylidenediphenol), which is useful as a raw material for producing polycarbonates and epoxy resins.

The copolymer beads used to make ion-exchange resins are preferably spherical beads, and a uniform bead size throughout a particular batch of copolymer is desirable because it produces uniform, predictable hydraulic properties, such as flow rate and pressure drop, for a bed of the resin in a reaction vessel. Suspension polymerization, in which water-insoluble monomers are suspended and polymerized as discrete droplets in an aqueous medium, inherently produces beads that are generally spherical. The size of the beads depends upon the size of the monomer droplets that form, and various techniques are used to control the diameter and uniformity of the droplets. Additives are used in the aqueous phase to help control droplet size by varying the interfacial tension between the monomer and the aqueous medium; they also are used to limit the growth by coalescence of the monomer droplets. The intensity of agitation is also varied to help control the droplet size. Suspension polymerization has been used for over half a century to produce the copolymer intermediates for ionexchange resins, as is disclosed for example by Boyer in U.S.-A-2,500,149. One technique that has been used to increase the uniformity of the droplet size is jetting a stream of monomer through an accurately sized orifice into the aqueous phase, for example the process disclosed by Koestler *et al.* in U. S.-A-3,922,255.

We have now found that strongly acidic cation-exchange resin beads, produced from crosslinked, styrenic copolymer beads which were formed by jetting the mixture of styrenic and crosslinker monomers into an aqueous liquid, and subsequently polymerizing the mixture, produce surprisingly high reaction rates when used to catalyze condensation reactions.

According to the present invention there is provided a process for catalyzing acid-catalyzed condensation reactions between reactants, which comprises contacting the reactants with jetted, suspension-polymerized styrenic copolymer beads functionalized with strongly acidic cation-exchange groups.

The reactants may, for example, comprise a mercaptan reaction promoter, e.g. in an amount of from about 1 to about 40% by weight of the total reactants.

The temperature at which the reactants contact the styrenic copolymer beads may, for example, be from about 40°C to about 100°C.

In the acid-catalyzed condensation of phenol with aldehydes or ketones, the process of the present invention results in a surprisingly greater conversion of the aldehyde or ketone to the condensation product, compared with the conversion achieved with non-jetted, batch-polymerized cation-exchange resin beads.

The styrenic copolymer beads used in the present invention may, for example, be prepared by a process which comprises the steps of:

(a) jetting a mixture of one or more styrenic monomers, one or more crosslinking monomers and a free-radical polymerization initiator into a moving, aqueous suspending medium to form uniformly sized monomer droplets,

(b) heating the droplets to a temperature above the activation temperature of the polymerization initiator until the droplets polymerize,

(c) separating the resulting polymer beads from the suspending medium,

(d) drying the beads, and

(e) functionalizing the beads with strongly acidic cation-exchange groups.

In the above process for producing the styrenic copolymer beads at least 50 percent by weight of the monomer mixture may, for example, be styrene.

Also in the above process for producing the styrenic copolymer beads the polymerization initiator may, for example, have an activation temperature of at least about 40°C.

Styrenic monomers, useful in preparing the crosslinked copolymer beads used in the present invention, include styrene and substituted styrenes such as α-methylstyrene, vinyltoluene, ethylvinylbenzene, vinylnaphthalene and the like.

Crosslinking monomers, useful in preparing the crosslinked copolymer beads used in the present invention and containing a plurality of ethylenically unsaturated functional groups, include: aromatic crosslinking monomers such as divinylbenzene, divinyltoluene, trivinylbenzene, divinylchlorobenzene, diallyl phthalate, divinylnaphthalene, divinyl xylene, divinylethylbenzene, trivinyl naphthalene and polyvinylanthracenes; and aliphatic crosslinking monomers such as di- and polyacrylates and methacrylates exemplified by trimethylolpropane trimethacrylate, ethylene glycol dimethacrylate, ethylene glycol diacrylate, neopentyl glycol dimethacrylate, pentaerythritol tetra- and trimethacrylates, and trivinylcyclohexane. The crosslinking monomer is preferably present at levels from about 0.1% to about 20 weight percent of the total monomer, and more preferably from about 1% to about 10 weight percent of the total monomer. Preferred crosslinking monomers are aromatic crosslinking monomers, and particularly preferred is divinylbenzene.

In one embodiment of the present invention the styrenic copolymer is a copolymer polymerized from at least about 50 weight percent styrene and an aromatic crosslinking monomer.

A jetting suspension-polymerization process useful for forming the crosslinked copolymer beads used in the present invention is exemplified by, but not limited to, the process disclosed by Koestler et al. in U.S.-A-3,922,255. In that process a minimal solubility of the monomers in the aqueous suspending medium is important. Solubility can be decreased by adding an electrolyte to the aqueous suspending medium.

The jetting process produces monomer droplets in the suspending medium whose average diameter for the droplet population is preferably varied over the range from about 20 μm to about 1 mm, and the resulting copolymer beads may be produced with an average diameter for the bead population which varies over the same range. The jetting suspension-polymerization process produces a droplet size distribution that is narrow, resulting in uniformly sized droplets and uniformly sized copolymer beads.

Jetting, as disclosed in, for example, U.S.-A-3,922,255, is a method of suspension polymerization of vinyl monomers in which a mixture containing one or more of a monovinyl and/or polyvinyl monomer, and a polymerization initiator, is combined with a monomer-immiscible aqueous solution or suspension of a stabilizer by jetting the monomer mixture through capillary openings to form droplets in the aqueous phase.

The monomers may be jetted by themselves, or mixed with inert liquids or prepolymers which are dissolved in the monomers or formed by prepolymerization of the monomers, or by a combination of both methods. The preferred jetting rate produces a weight ratio of suspending medium to monomer of from about 1.5:1 to about 10:1, and more preferably from about 2:1 to about 5:1. The monomer may be jetted into the suspending medium at a temperature above the activation temperature of the free-radical polymerization initiator, described below, which will cause polymerization to begin almost immediately, or the medium may be below the activation temperature, but preferably above about 15°C, and be heated subsequently, after flowing into a heating zone; this will permit the monomer droplets to stabilize before polymerization begins. Preferably, the monomer is jetted into the suspending medium at a temperature of from about 15°C to about 100°C.

All commonly used stabilizers, especially gelatin, starch, carboxymethylcellulose, polyacrylic acids, polyvinyl alcohol; or water-insoluble inorganic stabilizers in particulate form, such as bentonite, magnesium hydroxide and the like; or combinations of such stabilizers may be used to stabilize the monomer droplets in this or other jetting suspension-polymerization processes.

The usual initiators for radical polymerization (organic peroxides, redox systems, etc.) may be used to start the reaction. Free-radical polymerization initiators are preferred to initiate polymerization of the monomer droplets suspended in the suspending medium. Preferred free-radical polymerization initiators are oil-soluble initiators which are dissolved in the monomer, such as benzoyl peroxide, lauroyl peroxide, t-butyl peroctoate, t-butylperoxybenzoate, t-butylperoxypivalate, t-butylperoxy-2-ethylhexanoate, bis(4-t-butylcyclohexyl) peroxydicarbonate and the like; and azo compounds such as azobisisobutyronitrile, azobis-dimethylvaleronitrile and the like. The polymerization temperature, that is, the temperature at which the suspending medium is held during polymerization of the monomer droplets, and the polymerization initiator are interdependent in that the temperature must be high enough to break the chosen initiator down into an adequate number of free radicals to initiate and sustain polymerization, that is, it must be above the activation temperature of the initiator. Preferred polymerization temperatures are from about 40°C to about 100°C, and more preferably from about 50°C to about 90°C, and the free-radical initiator is chosen so that it has an activation temperature below the polymerization temperature. If polymerization is conducted under pressure, even higher temperatures can be used.

In one method for preparing jetted copolymer beads, for use in preparing the catalyst used in the present invention, the monomers and initiator are mixed and introduced into a droplet-formation column near its base through a plurality of orifices which may range in diameter generally from 0.1 to 0.4 mm in diameter, but which are uniform in size for any given polymerization. Most conveniently, the device used is one or more perforated discs with the holes spaced in such a manner as to not disturb the uniformity of the droplets being formed.

The size of the droplets formed is dependent on:

a. the diameter of the orifices,

b. the monomer flow rate through the orifices,

c. the difference in the physical properties: viz. viscosity, density, and surface tension, of the aqueous and monomeric media, and

d. the material from which the orifices are formed.

The orifices may be formed through metal, glass, plastic, or even rubber. It is noted that the surface tension of the material affects the jet velocity through the orifice.

The diluted aqueous medium is introduced near the base of the droplet formation column in such a manner as to minimize turbulence. The aqueous medium is introduced into the droplet formation column in close proximity to the drop-forming filaments of monomer. At this point the aqueous medium stabilizes the monomer droplets as they are formed.

Continuous introduction of the aqueous phase to the droplet formation column is preferable to provide fresh medium to stabilize the droplets as they are formed. The buoyant, newly-formed drops, which are relatively uniform in size, rise through the droplet formation column by virtue of their buoyancy. The aqueous medium which is introduced near the bottom also flows out the top of the droplet formation column.

The top of this column is tapered in such a way that turbulence which would alter the drop uniformity is avoided. This is accomplished by means which are readily known to those skilled in the art of designing flow through conduits.

The mixture of stabilized monomer droplets and aqueous medium is introduced into the upper part of the polymerization initiation column. The polymerization initiation column is maintained in the range of 50° - 90°C., thereby providing the proper temperature for the initiation of the polymerization of the monomers.

The proper temperature in the polymerization initiation column can be maintained by the introduction of an aqueous stream entering at the top of the polymerization initiation column. This aqueous stream combines with the aqueous stream from the droplet formation column after it enters the polymerization initiation column, and the combined aqueous stream exists from the bottom of the polymerization initiation column. The velocity of the aqueous stream is maintained such that it overcomes the buoyant force of the polymerizing monomer drops and imparts a small net downward velocity to the monomer droplets. By suitable adjustments of the aqueous velocity, precise control of the droplet residence time is achieved.

The polymerization initiation column is operated in such a manner that the efflux from the bottom of the column is a mixture of now partially polymerized monomer drops and aqueous medium. Alternatively, the monomer/initiator mixture and the aqueous medium may be introduced into the upper end of the droplet formation column, and the droplets flowed from the bottom thereof to the polymerization initiation column.

The height of the polymerization initiation column is such that by suitable adjustment of the aqueous flow velocity therethrough and the aqueous medium temperature, the desired degree of polymerization can be achieved in the droplets which flow from the bottom of the polymerization initiation column. It is highly preferable to achieve such a degree of polymerization therein, such that the droplets will not change their shape or size when subjected to shear stresses commonly experienced in moderately agitated systems.

The slurry of partially polymerized droplets in aqueous medium flows from the bottom of the polymerization initiation column to a suitably designed separation vessel. In this separation vessel advantage is taken of the buoyant nature of the partially polymerized droplets and the slurry is concentrated from 8-30% droplets up to 40-60% droplets. The concentrated slurry flows from the top of the separation vessel, whereas the aqueous medium, now essentially free of droplets, is allowed to flow from the bottom of the separation vessel. The top of the separation vessel is conical in nature, to facilitate the concentration of the droplets or beads. The volume of the separation vessel is such that there is sufficient residence time for the concentration of the beads to occur.

The separated aqueous liquid from the separation vessel flows from the bottom of said vessel to a recycle tank. In the recycle tank the aqueous liquid is accumulated for recycle to the polymerization initiation column. Extraneous solids are removed in this recycle tank by suitable techniques well known to the art. The aqueous liquid returns through a conduit into the top of the polymerization initiation column for recycle. On its passage through the conduit the aqueous recycle is either heated or cooled, as required, to maintain the proper temperature range in the polymerization initiation column previously described.

The concentrated bead slurry flowing from the top of the separation vessel is led to a bank of agitated reactors, wherein the polymerization of the droplets is completed. Each reactor is operated batch style, first by introducing an amount of aqueous suspension mixture capable of being agitated, and then an appropriate amount of concentrated droplet slurry such as to make full use of the reactor volume available. When the reactor is filled by use of the aforementioned technique the temperature is varied in such a manner as to complete

EP 0 486 277 B1

the polymerization.

It is essential that the beads entering the reactor be sufficiently polymerized so as to maintain their size and shape during the completion of the polymerization. If the drops are not sufficiently polymerized the generation of a large amount of very small polymer particles will occur in the reactor.

After completion of the polymerization, the bead slurry may pass to an optionally provided slurry holding vessel, or directly to a conventional dewatering device. Although the beads or particles size is greatly influenced by orifice size and other factors, generally the size will vary from 0.20 to 2 millimeters in diameter. It has also been found desirable to space the orifices some distance apart. Understandably these distances may vary considerably depending on orifice size, flow rates, etc. A minimum spacing of 2.03 mm (0.08 inch) from center to center is however preferred. Similarly a jet velocity of 0.25 to 10.00 cc/minute/orifice would most conveniently be applied, with preferred rates for each orifice size.

Other processes which form uniformly sized copolymer beads by jetting monomer into an aqueous suspending liquid may be used, for example that disclosed by Timm *et al.* in U.S.-A-4,623,706, which uses a vibrating orifice to jet the monomer into the suspending medium. The suspending medium preferably moves with relation to the jetting orifice or orifices, and the monomer droplets may either be polymerized in the vicinity of the orifices by jetting the monomer into the suspending medium at the polymerization temperature, or they may be polymerized in a different zone of the polymerization apparatus by causing the moving suspending medium to carry them into a heated polymerization zone. The polymerized beads may be separated from the suspension medium by gravity, by centrifugal flow, by hydraulic separation or by filtration.

Functionalization processes known to those skilled in the art may be used to functionalize the jetted copolymer beads with strong-acid functionality. The preferred strong-acid functionality is the sulfonic acid group, and known processes for sulfonating the copolymer may be used, including sulfonation processes which monosulfonate the aromatic ring and those which substitute the aromatic ring with more than a single sulfonic acid group. The preferred sulfonation produces a strongly acidic cation-exchange resin with a cation-exchange capacity of from about 4.8 to about 5.4 milliequivalents per gram (dry basis) and a moisture-holding capacity of from about 60 to about 70%. Moisture-holding capacity, as used herein, refers to the amount of water a functionalized copolymer will retain, and is determined by weighing a drained but wet, functionalized copolymer sample, then drying the sample at mild conditions, e.g. 100-110°C and atmospheric pressure, to a constant weight, and reweighing the sample. Moisture-holding capacity is calculated as this weight difference, expressed as a percentage of the dried sample weight.

In accordance with the present invention, the reactions catalyzed by the strongly acidic cation-exchange resin beads are condensation reactions, for example, the condensation of phenols with ketones or aldehydes to produce bisphenols. A preferred reaction which is catalyzed by the strongly acidic ion-exchange resin beads according to the present invention is the reaction of phenol with acetone. In this preferred reaction the ratio of phenol to acetone is preferably from about 20:1 to about 2:1. More preferred is that reaction in which phenol and acetone are combined in a molar ratio of from about 20:1 to about 2:1 and the combination is contacted, at from about 40°C to about 100°C, with from about 1 to about 40 weight percent (based on the weight of phenol and acetone) of the strongly acidic ion-exchange resin beads of the present invention, optionally in the presence of from about 1 to about 40 weight percent (based on the weight of phenol and acetone) of a mercaptan reaction promoter, preferably ethanethiol, aminoethane-thiol or dimethylthiazolidine.

In the process of the present invention, for catalyzing condensation reactions, the styrenic copolymer beads may, for example, be present in an amount of from about 1 to about 40% of the total weight of the reactants.

As is shown by the following Examples, use of the strongly acidic cation-exchange resin beads according to the present invention in the condensation of phenols with aldehydes or ketones produces a higher conversion of the reactants to bisphenols than can be obtained with strongly acidic cation-exchange resin beads produced from batch-suspension-polymerized copolymer beads formed without jetting the monomer into the suspending medium, and this higher conversion is achieved without sacrificing the selectivity of the reaction for bisphenols. Without wishing to be bound by theory, we believe that the higher conversion results from a higher reaction rate achieved with the resin beads used in the present invention. Given a long enough reaction time, the difference in conversion between jetted and non-jetted copolymer resin beads may disappear, but practical, commercial processes do not have unlimited time. The ability to produce more bisphenol product in a given time, which is afforded by a higher reaction rate in such processes, is an advantage that is readily apparent to those skilled in the art. The difference in reaction rate is all the more surprising because we are not aware of any theoretical basis for the difference between resin from jetted copolymer beads and that from beads formed without jetting.

The following Examples are presented to illustrate preferred embodiments of the present invention. All ratios and percentages are by weight, unless otherwise indicated, and all reagents are of good commercial quality

5

unless otherwise indicated. The catalysts used in the following Examples are designated Catalyst K to indicate a comparative catalyst produced by suspension polymerization in a kettle, and Catalyst J to indicate a catalyst according to the present invention produced by jetting suspension polymerization.

EXAMPLE 1

This Example illustrates the preparation of jetted copolymer beads useful in making strongly acidic, cation exchange resin beads according to the present invention.

An aqueous suspending medium was prepared containing 0.55% of Acrysol A-5 polyacrylic acid dispersant, 0.2% sodium hydroxide, 0.39% boric acid, 0.04% gelatin and 0.025% methylene blue, and having a pH of between 8.5 and 8.7. A monomer solution was prepared containing 7.3% commercial divinylbenzene (containing 55% pure divinylbenzene and 45% ethylvinylbenzene), 92.1% styrene, 0.3% t-butyl peroctoate and 0.3% bis(4-t-butylcyclohexyl) peroxydicarbonate. The monomer mixture was jetted through vibrating jetting orifices 450 μm in diameter, at a rate of 145 kg/hr, into a stream of the suspending medium moving at a rate of 386 liter/hr. This dispersion was conveyed by the flow of suspending medium to a gelling column held at 63°C. The flow produced a residence time of 3.5 hours in the gelling column, and the conversion of monomer to copolymer during this time was 25%. As the suspension of copolymer exited the gelling column, additional 0.6% aqueous methylene blue solution was added at a rate of 2.8 liter/hour. The copolymer was separated from the aqueous phase, which was recycled. The copolymer was then held in a finishing kettle for 4 hours at 65°C, then transferred to a final finishing kettle and held at 80°C for 1.5 hours, heated to 92°C, and held at that temperature for 1 hour. The finished copolymer was washed with water and air dried, for later sulfonation to Catalyst J.

EXAMPLE 2

This Example illustrates the preparation of the comparative copolymer beads by a batch process which does not involve jetting the monomer into the suspending medium.

An aqueous suspending medium of 355 g water, 1.12 g gelatin, 0.83 g boric acid, and 13.78 g poly(diallyldimethylammonium chloride) dispersant was prepared and adjusted to a pH between 10.0 and 10.5. A monomer mixture was prepared containing 340.0 g styrene, 30.0 g commercial divinylbenzene (containing 55% pure divinylbenzene and 45% ethylvinylbenzene) and 1.42 g tert-butyl peroctoate. The monomer mixture was stirred into the suspending medium in a reaction vessel to form a suspension of monomer droplets. This suspension was blanketed with nitrogen, heated to 75°C, and held at that temperature for 5 hours, then heated to 98°C and held at that temperature for 1 hour. The copolymer was then washed with water and air dried for later sulfonation to Catalyst K.

EXAMPLE 3

This Example illustrates preparation of strongly acidic, cation-exchange resin beads from the copolymer beads of Examples 1 and 2.

In a glass lined vessel was mixed 190 g of copolymer from Example 1 or Example 2, 1450 g sulfuric acid (96.6%) and 65 g ethylene dichloride. This mixture was heated to 130°C, held at that temperature for 15 minutes and cooled to 120°C. The sulfonated resin was hydrated at a temperature between 95°C and 110°C by consecutive additions of water and removal of a volume of the resulting, diluted acid equal to the water added, until the removed liquid was essentially neutral. The acid free material was then removed from the vessel and drained. The properties of the two resulting catalysts are shown in Table I below.

## Table I

| Catalyst | Cross-linker level | Cation Exchange Capacity | Moisture Holding Capacity | Bead Size |
|---|---|---|---|---|
| Catalyst K | 4% | 5.15 | 59.4% | 425 - 600 μm |
| Catalyst J | 4% | 5.10 | 60.2% | 425 - 600 μm |

## EXAMPLE 4

This Example illustrates the catalytic activity of the strongly acidic cation-exchange resins according to the present invention in catalyzing the condensation of phenol and acetone to bisphenol-A.

To flasks containing 90 grams of 99+% phenol were added 10 grams (10% by weight) of dry catalyst K and catalyst J, respectively. The temperature was raised to, and held at, 75°C and the phenol-catalyst mixture was stirred for one hour. Ten-milliliter portions of acetone (equivalent to a 7:1 phenol:acetone molar ratio) were added to each flask and the reaction was monitored as it proceeded at 75°C by periodically removing 1-ml samples, quenching them with a water/methanol mixture and then analyzing them by high-pressure liquid chromatography. Acetone conversion is calculated as the area of the bisphenol-A peaks (both the ortho and para isomers), expressed as a percentage of the total area of the acetone peak and the bisphenol product peaks. Selectivity to bisphenol-A is calculated as the area of the bisphenol-A peaks (both the ortho and para isomers), expressed as a percentage of the total area of bisphenol-A peaks and those of all other reaction products of phenol and acetone. These results are presented in Table II, below.

### Table II

| Catalyst | Time | Acetone Conversion | Selectivity to BPA |
|---|---|---|---|
| Catalyst K | 1 hr | 54.6% | 85.3% |
| Catalyst J | 1 hr | 60.1% | 85.5% |

## EXAMPLE 5

This Example illustrates the effectiveness of the strongly acidic cation-exchange resins according to the present invention in the presence of mercaptan reaction promoters.

To flasks containing 90 grams of 99+% phenol were added 10 grams (10% by weight) of dried catalysts K and J, respectively. Each catalyst used in this Example had a particle size between 425 and 600 μm, and each had been treated with 7.5 millimoles of aminoethanethiol reaction promoter. The temperature was raised to, and held at, 75°C and the flask contents were stirred for one hour. Ten-milliliter portions of acetone (equivalent to a 7:1 phenol:acetone molar ratio) were added to each flask, and the reaction was monitored as it proceeded at 75°C by periodically removing 1-ml samples, quenching with a water/methanol mixture and then analyzing by high-pressure liquid chromatography. The resulting acetone conversion and selectivity to bisphenol-A, determined as described in Example 4, are presented in Table III, below.

### Table III

| Catalyst | Time | Acetone Conversion | Selectivity to BPA |
|---|---|---|---|
| Catalyst KP | 1 hr | 80.2% | 97.3% |
| Catalyst JP | 1 hr | 88.4% | 96.9% |

## Claims

1. A process for catalyzing condensation reactions between reactants, which comprises contacting the reactants with jetted, suspension-polymerized styrenic copolymer beads functionalized with strongly acidic cation-exchange groups.

2. A process as claimed in claim 1, wherein the reactants comprise phenol and an aldehyde or ketone.

3. A process as claimed in claim 2, wherein said aldehyde or ketone is acetone.

4. A process as claimed in claim 3, wherein the ratio of phenol to acetone is from about 20:1 to about 2:1.

5. A process as claimed in any preceding claim, wherein the reactants comprise a mercaptan reaction promoter.

6. A process as claimed in claim 5, wherein the mercaptan reaction promoter is present in an amount of from about 1 to about 40% by weight of the total reactants.

7. A process as claimed in any preceding claim, wherein the beads are present at from about 1% to about 40% of the total weight of the reactants.

8. A process as claimed in any preceding claim, wherein the temperature at which the reactants contact the beads is from about 40°C to about 100°C.

9. A process as claimed in any preceding claim, wherein the styrenic copolymer is a copolymer polymerized from at least about 50 weight percent styrene and an aromatic crosslinker.

10. A process as claimed in claim 9, wherein the aromatic crosslinker is divinylbenzene.

11. A process as claimed in claim 9 or claim 10, wherein the aromatic crosslinker is present at from about 1% to about 20% by weight of the copolymer.

12. A process as claimed in any preceding claim, wherein the styrenic copolymer beads are prepared by a process which comprises the following steps:
    (a) jetting a mixture of one or more styrenic monomers, one or more crosslinking monomers and a free-radical polymerization initiator into a moving, aqueous suspending medium to form uniformly sized monomer droplets,
    (b) heating the droplets to a temperature above the activation temperature of the polymerization initiator until the droplets polymerize,
    (c) separating the resulting polymer beads from the suspending medium,
    (d) drying the beads, and
    (e) functionalizing the beads with strongly acidic cation-exchange groups.

13. A process as claimed in claim 12, wherein at least 50% by weight of the monomer mixture is styrene.

14. A process as claimed in claim 12 or claim 13, wherein the polymerization initiator has an activation temperature of at least about 40°C.

15. A process as claimed in any of claims 12 to 14, wherein the average diameter of the polymer droplet population is from about 20 μm to about 1 mm.

16. A process as claimed in any of claims 12 to 15, wherein the monomer mixture is jetted into the suspending medium at a ratio of suspending medium to monomer mixture of from about 1.5:1 to about 10:1.

17. A process as claimed in claim 16, wherein the ratio of suspending medium to monomer mixture is from about 2:1 to about 5:1.

18. A process as claimed in any of claims 12 to 17, wherein the monomer mixture is jetted into the suspending medium at a temperature from about 15°C to about 100°C.

**Patentansprüche**

1. Verfahren zur Katalysierung von Kondensationsreaktionen zwischen Reaktionsmitteln, bei dem die Reaktionsmittel mit gespritzten, Suspensions-polymerisierten Styrol- Copolymerkügelchen in Kontakt gebracht werden, die mit stark sauren Kationenaustauschergruppen funktionalisiert sind.

2. Verfahren nach Anspruch 1, worin die Reaktionsmittel Phenol und ein Aldehyd oder Keton umfassen.

3. Verfahren nach Anspruch 2, worin das Aldehyd oder Keton Aceton ist.

4. Verfahren nach Anspruch 3, worin das Verhältnis von Phenol zu Aceton etwa 20:1 bis etwa 2:1 ist.

5. Verfahren nach einem der vorhergehenden Ansprüche, worin die Reaktionsmittel einen Mercaptan-Reaktionsverstärker umfassen.

6. Verfahren nach Anspruch 5, worin der Mercaptan-Reaktions-verstärker in einer Menge von etwa 1 bis etwa 40 Gew.-% der gesamten Reaktionsmittel vorliegt.

7. Verfahren nach einem der vorhergehenden Ansprüche, worin die Kügelchen etwa 1 bis etwa 40 % des Gesamtgewichts der Reaktionsmittel betragen.

8. Verfahren nach einem der vorhergehenden Ansprüche, worin die Temperatur, bei der die Reaktionsmittel mit den Kügelchen in Kontakt kommen, etwa 40 bis etwa 100°C beträgt.

9. Verfahren nach einem der vorhergehenden Ansprüche, worin das Styrol-Copolymer ein aus mindestens etwa 50 Gew.-% Styrol und einem aromatischen Vernetzer polymerisiertes Copolymer ist.

10. Verfahren nach Anspruch 9, worin der aromatische Vernetzer Divinylbenzol ist.

11. Verfahren nach Anspruch 9 oder 10, worin der aromatische Vernetzer zu etwa 1 bis etwa 20 Gew.-% des Copolymers vorliegt.

12. Verfahren nach einem der vorhergehenden Ansprüche, worin die Styrol-Copolymerkügelchen durch ein Verfahren hergestellt werden, umfassend die folgenden Verfahrens- schritte:
    (a) Spritzen eines Gemisches ein oder mehrerer Styrol-Monomere, ein oder mehrerer Vernetzungsmonomere und eines Radikal-Polymerisationsinitiators in ein sich bewegendes, wäßriges Suspensionsmedium, wodurch gleich große Monomer-Tröpfchen gebildet werden,
    (b) Erhitzen der Tröpfchen auf eine Temperatur oberhalb der Aktivierungstemperatur des Polymerisationsinitiators bis die Tröpfchen polymerisieren,
    (c) Abtrennen der erhaltenen Polymer-Kügelchen von dem Suspensionsmedium,
    (d) Trocknen der Kügelchen, und
    (e) Funktionalisieren der Kügelchen mit stark sauren Kationenaustauschergruppen.

13. Verfahren nach Anspruch 12, worin mindestens 50 Gew.-% des Monomergemisches Styrol ist.

14. Verfahren nach Anspruch 12 oder 13, worin der Polymerisationsinitiator eine Aktivierungstemperatur von mindestens etwa 40°C hat.

15. Verfahren nach einem der Ansprüche 12 bis 14, worin der Durchschnittsdurchmesser der Polymer-Tröpfchenpopulation etwa 20 µm bis etwa 1 mm beträgt.

16. Verfahren nach einem der Ansprüche 12 bis 15, worin das Monomergemisch in das Suspensionsmedium in einem Verhältnis des Suspensionsmediums zu dem Monomergemisch von etwa 1,5:1 bis etwa 10:1 gespritzt wird.

17. Verfahren nach Anspruch 16, worin das Verhältnis des Suspensionsmediums zu dem Monomergemisch etwa 2:1 bis etwa 5:1 beträgt.

18. Verfahren nach einem der Ansprüche 12 bis 17, worin das Monomergemisch in das Suspensionsmedium bei einer Temperatur von etwa 15 bis etwa 100°C gespritzt wird.

**Revendications**

1. Procédé de catalyse de réactions de condensation entre des réactifs, ledit procédé comprenant la mise en contact des réactifs avec des perles projetées de copolymère styrénique polymérisé en suspension fonctionnalisé par des groupes échangeurs de cations fortement acides.

2. Procédé selon la revendication 1, dans lequel les réactifs comprennent du phénol et un aldéhyde ou une cétone.

3. Procédé selon la revendication 2, dans lequel ledit aldéhyde ou ladite cétone est l'acétone.

4. Procédé selon la revendication 3, dans lequel le rapport du phénol à l'acétone est d'environ 20:1 à environ 2:1.

5. Procédé selon l'une quelconque des revendications précédentes, dans lequel les réactifs comprennent un promoteur réactionnel à base de mercaptan.

6. Procédé selon la revendication 5, dans lequel le promoteur réactionnel à base de mercaptan est présent en quantité d'environ 1 à environ 40 % en poids de la quantité totale de réactifs.

7. Procédé selon l'une quelconque des revendications précédentes, dans lequel les perles sont présentes en quantité d'environ 1 à environ 40 % du poids total des réactifs.

8. Procédé selon l'une quelconque des revendications précédentes, dans lequel la température à laquelle les réactifs viennent en contact avec les perles est d'environ 40 à environ 100°C.

9. Procédé selon l'une quelconque des revendications précédentes, dans lequel le copolymère de styrène est un copolymère polymérisé à partir d'au moins environ 50 % en poids de styrène et d'un agent de réticulation aromatique.

10. Procédé selon la revendication 9, dans lequel l'agent de réticulation aromatique est le divinylbenzène.

11. Procédé selon la revendication 9 ou 10, dans lequel l'agent de réticulation aromatique est présent en quantité d'environ 1 à environ 20 % en poids du copolymère.

12. Procédé selon l'une quelconque des revendications précédentes, dans lequel les perles de copolymère styrénique sont préparées par un procédé qui comprend les stades suivants :
(a) on projette un mélange d'un ou plusieurs monomères styréniaues, d'un ou plusieurs monomères de réticulation et d'un initiateur de polymérisation à radicaux libres dans un milieu de suspension aqueux mobile pour former des gouttelettes monomeres calibre uniforme,
(b) on chauffe les gouttelettes à une température supérieure à la température d'activation de l'initiateur de polymérisation jusqu'à ce que les gouttelettes se polymérisent,
(c) on sépare les perles de polymère obtenues du milieu de suspension,
(d) on sèche les perles, et
(e) on fonctionnalise les perles par des groupes échangeurs de cations fortement acides.

13. Procédé selon la revendication 12, dans lequel au moins 50 % en poids du mélange de monomères sont constitués de styrène.

14. Procédé selon la revendication 12 ou 13, dans lequel l'initiateur de polymérisation a une température d'activation d'au moins 40°C environ.

15. Procédé selon l'une quelconque des revendications 12 à 14, dans lequel le diamètre moyen de la population de gouttelettes polymères est d'environ 20 micromètres à environ 1 mm.

16. Procédé selon l'une quelconque des revendications 12 à 15, dans lequel le mélange de monomeres est projeté dans le milieu de suspension selon un rapport du milieu de suspension au mélange de monomères d'environ 1,5:1 à environ 10:1.

17. Procédé selon la revendication 16, dans lequel le rapport du milieu de suspension au mélange de monomères est d'environ 2:1 à environ 5:1.

18. Procédé selon l'une quelconque des revendications 12 à 17, dans lequel le mélange de monomères est projeté dans le milieu de suspension à une température d'environ 15 à environ 100°C.